# EUROPEAN PATENT APPLICATION

(11) **EP 4 218 925 A1**
(43) Date of publication of application: **02.08.2023**
(21) Application number: 23150740.1
(22) Date of filing: 09.01.2023
(51) Int. Cl.: A61P 1/04, A23L 29/30, A23L 33/135, A23L 33/125, A23L 33/105

(54) **DIETARY SUPPLEMENT FOR THE TREATMENT OF GASTROESOPHAGEAL REFLUX**

(30) Priority: 26.01.2022 IT 202200001271
(71) Applicant: Geophyt S.r.L., 15067 Novi Ligure (AL) (IT)
(72) Inventor: MERLO, Enrico, 15067 Novi Ligure (Alessandria) (IT)
(74) Representative: Lunati & Mazzoni S.r.L.

(57) **Abstract**

It is provided a dietary supplement for the treatment of gastroesophageal reflux comprising maltodextrin, hyaluronic acid, Aloe vera, Klamath algae and Mastiha resin.

## Description

The present invention relates to a dietary supplement for the treatment of gastroesophageal reflux of the type specified in the preamble to the first claim.

In particular, the present invention relates to a dietary supplement for soothing inflammation with tearing of tissues mainly affecting the upper stomach and oesophagus.

As is well known, gastroesophageal reflux is typically caused by the reflux of stomach contents and intestine-produced gas into the oesophagus, resulting in duodenal-gastroesophageal reflux.

Other causes of reflux can derive from a decrease in the tone of the cardia, i.e. the lower oesophageal sphincter acting as a valve separating the oesophagus from the stomach, due to the intake of particular substances such as fatty foods, nicotine, caffeine, citrus fruits, alcohol, certain types of medication or, in rarer cases, due to prolonged stagnation of the bolus in the stomach, due to dyskinesias that slow down its normal emptying; or even the intake of excessively large meals; of course, physical conditions that lead to increased gastric pressure, such as obesity and pregnancy, can also play a role.

In any case, during reflux, the hydrochloric acid and the bile come into contact with the mucous membrane of the oesophagus causing inflammation, also known as oesophagitis, with the possible onset of characteristic symptoms such as the pyrosis.

Thus, over time, the inflammation may evolve into damage to the tissue of the oesophagus, in the form of erosions and small ulcers.

Various drugs and/or substances are currently used for the treatment and cure of gastroesophageal reflux. These drugs are mainly aimed at reducing ulcer-related effects on the inner surface of the oesophagus.

Such drugs or substances may include the proton pump inhibitors or PPIs, sucralfate, propolis, maalox and gaviscon.

The known technique described includes some major drawbacks.

In particular, none of the above-mentioned substances can really overcome the oesophageal inflammation resulting from reflux.

In detail, PPIs reduce the acidity of gastric juices over a prolonged period of time to prevent or slow down the ulcerating effects, but has no soothing purpose to reduce the unpleasant effects of ongoing ulcers or lesions.

The same applies to sucralfate which is a class C drug whose function is not to soothe or cure ulcers, but to isolate them from gastric juices from the stomach or other painful acid substances.

The propolis, on the other hand, is a basically ineffective palliative.

The Maalox is not intended to intervene in the pathology, but is simply an anti-acid drug that, like PPIs, inhibits the production of acid substances within the stomach.

In conclusion, also the Gaviscon also works mainly on the gastric juices inside the stomach and intervenes by creating a protective veil over the liquid in the stomach so that gastric acids do not rise to areas where ulcers are present. This drug therefore has no effect on the pathology in question.

In this situation, the technical task underlying the present invention is to devise a dietary supplement for the treatment of gastroesophageal reflux that can substantially obviate at least some of the aforementioned drawbacks.

In the context of this technical task, it is an important aim of the invention to obtain a dietary supplement that is capable of inhibiting inflammation of the tissues of the upper stomach and oesophagus.

In particular, an important purpose of the invention is to make a supplement that can also inhibit the occurrence of lesions in the areas affected by inflammation. Another important aim of the invention is to create a supplement to alleviate the pain resulting from the onset of inflammation and injuries caused by reflux. Furthermore, a further task of the invention is to realise a dietary supplement with high healing capabilities.

In conclusion, another important aim of the invention is to realise an integrator that is both minimally invasive, and thus able to improve user compliance, and cost-effective.

The technical task and specified purposes are achieved by a dietary supplement for the treatment of gastroesophageal reflux disease as claimed in the annexed claim 1.

Preferred technical solutions are highlighted in the dependent claims.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as performed in the International Standard Atmosphere ICAO (ISO 2533:1975).

The invention relates to a dietary supplement for the treatment of gastroesophageal reflux, in particular a dietary supplement intended primarily for the treatment of effects resulting from inflammation of tissues in the area of the upper stomach and oesophagus, for example in the vicinity of the cardia.

In detail, the dietary supplement for the treatment of gastroesophageal reflux is designed to soothe pain arising from tissue inflammation and to promote the healing and healing of tissue lesions that may arise from the inflammation.

The dietary supplement can be an aqueous solution. However, preferably, the dietary supplement is in the form of a powder granulate.

More particularly, the invention allows for at least one stick pack comprising a predetermined dose of powdered supplement. More in detail, preferably, the stick pack comprises a dose of powdered supplement.

For example, a recommended dose may be about 2 g.

The dietary supplement for the treatment of gastroesophageal reflux according to the invention preferably comprises five main active components.

These main active components are a first component consisting of maltodextrin and, advantageously, a second component consisting of hyaluronic acid, a third component consisting of aloe vera, a fourth component consisting of Klamath algae and a fifth component consisting of Mastiha resin.

The maltodextrin is basically a water-soluble complex carbohydrate. Typically, it is obtained by chemical hydrolysis processes mainly from the breakdown of starches from cereals, e.g. maize, oats, wheat and rice, or tubers, e.g. potatoes and tapioca. Preferably, the supplement includes maltodextrin obtained from maize. An example of this product is commonly marketed under the brand name GLUCIDEX^{™}.

Furthermore, the maltodextrin is preferably in powder form.

The maltodextrin is therefore preferably present in doses of between 76% and 80% by weight of the total supplement weight.

More specifically, the maltodextrin may be present in doses of between 77% and 79% by weight of the total supplement weight.

Appropriately, maltodextrin may be present in a weight dose of 78% of the total supplement weight.

The hyaluronic acid is a non-sulphated glycosaminoglycan with no protein core of the unbranched polysaccharide chain produced by the condensation of thousands of disaccharide units formed at the same time by glucuronic acid and N-acetylglucosamine residues linked together alternately by β1→4 and β1→3 glycosidic bonds, as well as by intramolecular hydrogen bonds, which stabilise their conformations.

The hyaluronic acid has a high degree of hydration depending on the fact that it is able to complex with many water molecules, due to its solubility resulting from the high polarity of the hyaluronate molecule.

An example of hyaluronic acid is that marketed by: Eyepharma under the brand name Ialutec^{™}, or that marketed by: Ibsa pharmaceutics under the brand name Ialuril^{™}.

The supplement preferably comprises linear hyaluronic acid. Preferably, the supplement also comprises hyaluronic acid in powder form.

Thus, the hyaluronic acid is preferably present in doses of between 0.9% and 1.3% by weight of the total supplement weight.

More specifically, the hyaluronic acid may be present in doses of between 1% and 1.2% by weight of the total weight of the supplement.

Appropriately, the hyaluronic acid may be present in a dose of 1.1% by weight of the total supplement weight.

The aloe vera is a succulent plant of the Aloeaceae family that prefers warm, dry climates.

Generally, this plant is found in hot, dry climates and consists of tufted, simple leaves about 40-60 cm long.

The leaves are generally long lance-shaped, with a sharp apex, and have a very thick, fleshy cuticle due to the presence of aquiferous parenchyma within them. From the chemical point of view, the aloe vera comprises three major classes of components: the complex sugars, in particular glucomannans, most notably the acemannan, in the transparent inner gel with immuno-stimulating properties; anthraquinones in the coriaceous green part of the leaf with a strong laxative action; and a variety of other substances such as mineral salts, vitamins, amino acids, organic acids, phospholipids, enzymes, lignins and saponins.

In particular, the aloe vera is generally used for skin care and has anti-irritant functions. For this reason, it is often used in products such as cleansers, soaps, shampoos and other skin cosmetics.

The supplement therefore includes aloe vera as pure aloe or an extract of it.

The aloe vera is included in the dietary supplement preferably in granular form. In particular, aloe vera is preferably present in both dry extract and powder form. More specifically, aloe vera may consist of 50% dry extract and 50% powder.

However, it could also be further processed to create a gel component.

An example of aloe vera is that marketed by Zuccari under the brand name [aloevera]^{2 ™} or by Aboca under the brand name NeoBianacid^{™}.

The aloe vera is preferably present in doses of between 1.5% and 1.9% by weight of the total weight of the supplement.

More specifically, aloe vera may be present in doses of between 1.6% and 1.8% by weight of the total weight of the supplement.

Appropriately, aloe vera may be present in a dose of 1.7% by weight of the total supplement weight.

The Klamath algae consists of a collection of algae, generally green or blue in colour, belonging to the species Aphanizomenon flos-aquae. The name originates from the fact that these algae grow and are harvested from Klamath Lake located in the United States and, more precisely, in Oregon.

The harvesting of the algae generally takes place during warm periods and, afterwards, the plants are accumulated inside tanks and subjected to a heat treatment that brings them to temperatures close to 0°.

Following micro-filtration they are purified and freeze-dried for marketing

The dietary supplement preferably includes Klamath algae in powder form. In addition, the supplement could include pure Klamath algae or an extract of it.

An example of Klamath algae is that marketed by Geophyt S.r.l. under the brand name Proklama^{™}, or that marketed by Nutrigea under the brand name Klamin^{™}. Therefore, the Klamath algae is preferably present in doses of between 1.45% and 1.85% by weight of the total supplement weight.

More specifically, the Klamath algae can be present in doses of between 1.55% and 1.75% by weight of the total weight of the supplement.

Appropriately, the Klamath algae may be present in a weight dose of 1.65% of the total supplement weight.

The Mastiha resin is a substance extracted from the mastic tree (Pistacia lentiscus L.) and also known as the 'tear of Chios', as it is mainly found on the island of Chios. It consists of a latex that can flow from the stem of the mastic tree and condense into small droplets. The resin also dries to a brittle, translucent hard resin when in contact with air.

An example of Mastiha resin is that marketed by 'The Chios Mastiha Growers Association' under the brand name Mastiha capsules^{™}.

The Mastiha resin is also preferably included in the supplement in powder form. In addition, the supplement could include pure Mastiha resin or an extract of it.

The Mastiha resin is preferably present in doses of between 3% and 7% by weight of the total weight of the supplement.

More specifically, the Mastiha resin may be present in doses of between 4% and 6% by weight of the total weight of the supplement.

Appropriately, the Mastiha resin may be present in a dose by weight of 5% of the total weight of the supplement.

The dietary supplement may include additional components in addition to those already described.

For example, the dietary supplement also includes althea.

The althea is a plant belonging to the Malvaceae family and the genus Althaea. Widely used in cosmetics, due to its high mucilage content, althea has emollient, laxative and calming properties.

It can in fact be used to treat bronchial catarrhs and coughs, decongest the intestines, and as a cosmetic for reddened skin and furunculosis.

Preferably, the dietary supplement includes althea in the form of a dry extract. Preferably, althea is also in powder form.

In particular, althea is preferably present in doses of between 0.5% and 4.5% by weight of the total weight of the supplement.

More specifically, the althea may be present in doses of between 1.5% and 3.5% by weight of the total weight of the supplement.

Appropriately, althea may be present in a dose by weight of 2.5 per cent of the total weight of the supplement.

In addition, the dietary supplement also includes mallow.

The wild mallow (Malva sylvestris L.) is a plant belonging to the Malvaceae family. It is, in particular, a herbaceous plant with an erect, prostrate stem that can grow from 60 to 80 cm and has 5 to 7-lobed palmate leaves with an irregularly serrated margin.

The active ingredients of this plant are mainly found in its flowers and leaves, which are rich in mucilage and are mostly used for their emollient and bechic properties, in catarrhal forms of the first bronchial tract, and also contain potassium, calcium oxalate, vitamins and pectin.

Preferably, the dietary supplement includes mallow as a dry and hydro-glyceric extract from the leaves. In addition, preferably mallow is in powder form.

An example of Mallow is that marketed by Sanofi under the brand name Bonlax^{™}, or that marketed by: Aboca under the commercial brand name NeoBianacid^{™}.

In addition, the mallow is preferably present in doses of between 0.5% and 4.5% by weight of the total supplement weight.

More specifically, the mallow may be present in doses of between 1.5% and 3.5% by weight of the total weight of the supplement.

Appropriately, mallow may be present in a weight dose of 2.5 per cent of the total weight of the supplement.

Basically, therefore, mallow may be present in a dose by weight corresponding to the dose of althea.

The dietary supplement may also include an additional component.

In particular, the supplement may include banana.

The banana, as is well known, is a fruit that develops in the form of bunches from a plant, the banao, originating in countries with a tropical climate.

The banana is preferably included in the supplement in granular form.

In particular, the aloe vera is preferably present both in the form of powdered juice and as an aromatic powder. More specifically, the banana may consist of 53-55% juice powder and 45-47% aromatic powder.

The banana is present in doses of between 5.5% and 9.5% by weight of the total supplement weight.

More specifically, the banana may be present in doses of between 6.5% and 8.5% by weight of the total supplement weight.

Appropriately, the mallow may be present in a weight dose of 7.5 per cent of the total weight of the supplement.

In a preferred, but not exclusive form of embodiment, for example, the dietary supplement can be realised as follows:

| **Components per 2 g Stick Pack** | **Quantity [g]** |
|---|---|
| Maltodextrin | 1,561 |
| Hyaluronic acid | 0,022 |
| Aloe vera dry extract | 0,017 |
| Aloe vera powder | 0,017 |
| Klamath algae | 0,033 |
| Mastiha resin | 0,100 |
| Altea dry extract | 0,050 |
| Mallow dry extract | 0,050 |
| Banana juice powder | 0,080 |
| Ripe banana in aromatic powder | 0,070 |

The dietary supplement for the treatment of gastroesophageal reflux according to the invention achieves important advantages.

In fact, the dietary supplement is able to inhibit inflammation in tissues of the upper stomach and oesophagus.

The combined and synergetic effect of aloe vera, klamat algae and mastiha resin makes it possible to significantly reduce the sensation of pain, as all components have inhibiting effects in this respect.

However, in addition, each component makes a specific contribution not only to inhibiting a user's sense of pain, but also to inhibiting the onset of oesopharyngeal mucositis, as well as the known side effects of radiation therapies.

The aloe vera is a powerful healer that accelerates the process of filling cracks and lacerations caused by radiological side effects.

In addition, klamat algae blocks ionising radiation and therefore realises shielding effects against the radiation one is subjected to during radiological therapies.

The mastiha resin further enhances the anti-inflammatory effects and adds to the healing effects of aloe vera. In fact, it is deposited on the fissures to create a protective layer that facilitates the aloe vera and at the same time protects the laceration from possible attacks by acidic substances.

Therefore, the synergy of the components makes the supplement excellent for both of the aforementioned conditions by combining the necessary beneficial effects in one product.

The additional components also have effects that synergistically enhance the previous aspects. Indeed, hyaluronic acid increases tissue hydration, which, in contact with aloe, further accelerates healing.

The mallow also contributes to the humectant effects of the supplement.

Moreover, the supplement, being mainly made up of natural products with specific and synergistic effects, does not have the side effects of the previously described drugs of the known technique. Compared to the latter, moreover, the injury-reducing effects are combined with the pain-relieving action.

The invasiveness of the supplement is therefore very low, not least because the guaranteed effects are so high that limited use is required.

In particular, given that tissue tearing during reflux results in release of calcium ions that escape from the injured tissue, the stick pack conformation allows, during intake, the supplement to be mixed with saliva so that the ingredients that make up the product begin to hydrate, assuming the right consistency once the right amount of time has elapsed, e.g. the time it takes to swallow. Once swallowed, the product performs its full function at the level of the oesophagus and upper gullet and stomach, remaining in place for several hours, exploiting the electrostatic bond between the COO- acid terminal of the hyaluronic acid and the calcium ions that are released from the site affected by reflux.

Thus, the kneaded supplement is able to attach itself to the wound, greatly increasing its effectiveness and preventing any unintentional dispersion of the product into the body.

The invention is susceptible to variations within the inventive concept as defined by the claims.

Here, all details can be replaced by equivalent elements and the materials, shapes and sizes can be any.

## Claims

1. Dietary supplement for the treatment of gastroesophageal reflux containing maltodextrin and **characterised by the fact that** it additionally includes hyaluronic acid, aloe vera, Klamath algae and Mastiha resin.

2. Supplement according to claim 1, wherein said maltodextrin is present in doses by weight of between 76% and 80% relative to the total weight of said supplement.

3. Supplement according to any one of the preceding claims, wherein said hyaluronic acid is present in doses by weight of between 0.9% and 1.3% relative to the total weight of said supplement.

4. Supplement according to any one of the preceding claims, wherein said aloe vera is present in doses by weight of between 1.5% and 1.9% relative to the total weight of said supplement.

5. Supplement according to any one of the preceding claims, wherein said aloe vera is present either in the form of a dry extract or in the form of a powder.

6. Supplement according to any one of the preceding claims, wherein said Klamath alga is present in weight doses of between 1.45% and 1.85% of the total weight of said supplement.

7. Supplement according to any one of the preceding claims, wherein said Mastiha resin is present in weight doses of between 3% and 7% relative to the total weight of said supplement.

8. Supplement according to any one of the preceding claims, further comprising althea.

9. Supplement according to any one of the preceding claims, wherein said mallow is present in doses by weight of between 0.5% and 4.5% relative to the total weight of said supplement.

10. Supplement according to any one of the preceding claims, further comprising mallow.

11. Supplement according to any one of the preceding claims, wherein said mallow is present in doses by weight of between 0.5% and 4.5% relative to the total weight of said supplement.

12. Supplement according to any one of the preceding claims, further comprising banana.

13. Supplement according to any one of the preceding claims, wherein said banana is present in doses by weight of between 5.5% and 9.5% relative to the total weight of said supplement.

14. Supplement according to any one of the preceding claims, wherein said banana is present either in the form of juice powder or in the form of an aromatic powder.

15. Stick pack comprising a dose of a supplement according to any one of the preceding claims, in the form of a granulate powder.
